# EUROPÄISCHE PATENTANMELDUNG

(11) **EP 2 113 264 A2**
(43) Veröffentlichungstag der Anmeldung: **04.11.2009**
(21) Anmeldenummer: 09157616.5
(22) Anmeldetag: 08.04.2009
(51) Int. Cl.: A61L 31/10, A61L 31/14, A61L 33/12

(54) **Implantat umfassend eine Oberfläche mit verringerten Thrombogenen Eigenschatten**

(30) Priorität: 02.05.2008 DE 102008021894
(71) Anmelder: Biotronik VI Patent AG, 6341 Baar (CH)
(72) Erfinder: Borck, Alexander, 91086, Aurachtal (DE)
(74) Vertreter: Lindner-Vogt, Karin L.

(57) **Zusammenfassung**

Die vorliegende Erfindung betrifft Implantate für einen menschlichen oder tierischen Organismus umfassend eine Oberfläche mit verringerten thrombogenen Eigenschaften, Verfahren zur Herstellung der erfindungsgemäßen Implantate sowie Verwendung der erfindungsgemäßen Implantate zur Verringerung der Dosis und/oder der Konzentration der Verabreichung einer systemischen Begleitmedikation mit ein oder mehreren blutgerinnungshemmenden Wirkstoffen vor, während und/oder nach Implantateinsetzung in einen menschlichen oder tierischen Organismus.

## Beschreibung

### Gebiet der Erfindung

Die vorliegende Erfindung betrifft Implantate für einen menschlichen oder tierischen Organismus umfassend eine Oberfläche mit verringerten thrombogenen Eigenschaften, Verfahren zur Herstellung der erfindungsgemäßen Implantate sowie Verwendung der erfindungsgemäßen Implantate zur Verringerung der Dosis und/oder der Konzentration der Verabreichung einer systemischen Begleitmedikation mit ein oder mehreren blutgerinnungshemmenden Wirkstoffen vor, während und/oder nach Implantateinsetzung in einen menschlichen oder tierischen Organismus.

### Stand der Technik

Implantate sind Stoffe oder Teile, die zur Erfüllung bestimmter Ersatzfunktionen für einen begrenzten Zeitraum oder auf Lebenszeit in den menschlichen oder tierischen Körper eingebracht werden. Im Gegensatz zu Transplantaten bestehen Implantate aus künstlichem Material (Alloplastik). Es wird häufig nach medizinischen, plastischen oder funktionellen Implantaten unterschieden.

Medizinische Implantate haben die Aufgabe, Körperfunktionen zu unterstützen oder zu ersetzen. Je nach Funktion werden sie auch als implantierbare Prothese bezeichnet. Bekannte Vertreter sind z. B. Herzschrittmacher, Hirnschrittmacher bei Parkinson, Herzimplantate, Cochleaimplantate, Implantate in der Zahnmedizin, Stents und Implantate, die dazu dienen, ein Depot eines Arzneistoffes zu bilden sowie verschiedene Formen des Gelenkersatzes.

Plastische Implantate werden in der plastischen Chirurgie, z. B. zum Ersatz von zerstörten Körperteilen oder auch zur Veränderung von bestehenden Körperteilen verwendet.

Funktionelle Implantate dienen zur Überwachung von menschlichen oder tierischen Funktionen, indem z. B. RFID (radio frequency identicifaction)-Chips unter die Haut verpflanzt werden.

Anhand der Vielzahl der vorhandenen Implantattypen kann man erkennen, dass Implantate und deren Anwendung in der Medizin einen großen Stellenwert erlangt haben.

Da bei klassischen Behandlungsprinzipien, wie beispielsweise bei der systemischen Applikation von ein oder mehreren Wirkstoffen, zum Teil erhebliche Nebenwirkungen, wie beispielsweise bei der Tumorbehandlung, zu erwarten sind, gewinnt eine lokale, kontrollierte Wirkstofffreisetzung am Zielort oder in der Nähe davon zunehmende Bedeutung (Local-Drug-Delivery-Konzept; LDD-Konzept). Um diese lokale Applikation von Wirkstoffen durchführen zu können, werden insbesondere Implantatgrundkörper mit Wirkstoffen beschichtet, die entweder am Zielort oder in dessen Nähe in einen menschlichen oder tierischen Organismus implantiert werden und so lokal Wirkstoffe freisetzen. Diese klinisch etablierte Methode wird jährlich weltweit millionenfach angewendet und es ist zu erwarten, dass bei Berücksichtigung der demoskopischen Verschiebung innerhalb der Alterspyramide der Bedarf an neuen Werkstoffen sowie neuen Darreichungsformen wächst.

Im orthopädischen Bereich sind im Zusammenhang von endoprothetischen Implantaten Implantat-assoziierte Infektionen und thromboembolische Komplikationen bekannt. Thromboembolie ist ein akuter venöser oder arterieller Gefäßverschluss, der durch einen verschleppten Thrombus, der durch das Anhaften von Thrombozyten an die Oberfläche des Implantates entstehen kann, hervorgerufen wird. Die häufigsten Formen der Thromboembolie sind die Embolie, insbesondere die Lungenembolie.

Im Bereich der Herz-Kreislauf-Erkrankungen bieten minimalinvasive Therapieformen zur Aufweitung und Stabilisierung stenosierter Koronargefäße durch die Perkutane Transluminale Coronarangioplastie (PTCA) und die Stentimplantation eine sich immer weiter e-tablierende Behandlungsmethode. Als Spätkomplikation ist hier neben der Wiederverengung des Gefäßes nach Stentimplantation (sogenannte In-Stent-Restenose (ISR)) und der Gewebeinflammation vor allem das Risiko einer Thrombose zu nennen.

Anhand dieser Beispiele wird die Bedeutung der Verringerung des Thrombose- bzw. Thromboembolierisikos nach Implantateinsetzung deutlich. Um dies zu erreichen, wird derzeit eine Begleitmedikation von ein oder mehreren blutgerinnungshemmenden Wirkstoffen systemisch an den menschlichen oder tierischen Organismus, dem ein Implantat eingesetzt wurde, verabreicht. Als Goldstandard, d. h. die Begleitmedikation der Wahl, hat sich die sogenannte "dual anti-platelet therapy" herauskristallisiert, in der beispielsweise Acetylsalicylsäure und Clopidogrel^{®} als blutgerinnungshemmende Wirkstoffe systemisch verabreicht werden. Eine solche Begleitmedikation wird üblicherweise solange systemisch verabreicht, wie das Implantat im menschlichen oder tierischen Organismus ein Anhaften von Thrombozyten oder sonstigen Blutbestandteilen an die Oberfläche des Implantates hervorruft. Das bedeutet üblicherweise, dass über Monate bzw. Jahre oder sogar bis zum Tod des Organismus die Begleitmedikation beibehalten werden sollte, um das Thrombose-/Embolierisiko zu verringern.

Aufgrund von Wirkstoffeigenschaften der blutgerinnungshemmenden Wirkstoffe, insbesondere Acetylsalicylsäure und Clopidogrel^{®} sind zum Teil erhebliche Nebenwirkungen zu erwarten.

Bei Acetylsalicylsäure sind bei chronischen Überdosierungen vorwiegend zentralnervöse Störungen, auch bekannt als "Salicylismus", zu beobachten, wohingegen bei akuten Überdosierungen vor allem das Säure-Basen-Gleichgewicht im menschlichen oder tierischen Organismus zum Teil erheblich gestört wird, wobei eine anfängliche zentrale Hyperventilation in eine respiratorische Alkalose übergehen kann. Ein renaler Kompensationsversuch mit Alkaliurie kann zu Kalium- sowie Chlorid- und Wasserverlust (letzteres durch Erbrechen) führen. Verschiedenste Erscheinungsbilder sind hierbei zu beobachten, beispielsweise Ohrensausen, Übelkeit, Erbrechen, Seh- und Hörbeeinträchtigung, Kopfschmerzen, Schwindel und Verwirrtheit.

Bei Clopidogrel^{®} werden als nicht erwünschte Begleiterscheinungen vor allem Blutungen beobachtet, wobei insbesondere gastrointestinale Blutungen und andere Blutungen, wie Purpura/blaue Flecken/Blutergüsse und Nasenbluten häufig beobachtet werden. Weniger häufig werden Hämatome, Hämaturie und Augenblutungen und gelegentlich werden intrakranielle Blutungen beobachtet.

Bei der Kombination von Acetylsalicylsäure und Clopidogrel^{®} wird ein signifikant erhöhtes Risiko für leichte, schwere und andere Blutungen, überwiegend im gastrointestinalen Bereich oder Blutungen im Bereich von Punktionsstellen beobachtet, wobei herausgefunden wurde, dass die Rate der schweren Blutungen abhängig von der Acetylsalicylsäure-Dosis ist und im Verlauf der Therapie abnimmt (CURE Studie).

Sollten Implantatträger einen weiteren medizinischen Eingriff benötigen, vor allem bei zahnärztlichen Eingriffe oder weiteren chirurgischen Eingriffen, insbesondere im Bereich der Kardiologie, des Knie- und Hüftgelenkersatzes, dann sollte die systemische Begleitmedikation mit den blutgerinnungshemmenden Wirkstoffen unterbrochen werden, um die Blutungsrate während und nach dem jeweiligen Eingriff nicht zu verstärken. Dies führt jedoch dazu, dass das Thrombose-/Embolierisiko aufgrund des Implantates wieder ansteigt.

Eine Aufgabe der vorliegenden Erfindung befasst sich deshalb damit, einerseits die durch das Implantat selbst vermittelten Risiken, insbesondere das Thrombose-/Embolierisiko, und andererseits die durch die Begleitmedikation vermittelten Nebenwirkungen, insbesondere Blutungen, zu verringern.

### Zusammenfassung der Erfindung

Die vorliegende Aufgabe wird durch die Gegenstände der unabhängigen Ansprüche gelöst.

Ein erster Erfindungsgegenstand betrifft ein Implantat für einen menschlichen oder tierischen Organismus, **dadurch gekennzeichnet, dass** die Oberfläche des Implantates einen Benetzungswinkel von Θ ≤ 80° hat.

Ein zweiter Erfindungsgegenstand betrifft ein Verfahren zur Herstellung eines erfindungsgemäßen Implantates, **dadurch gekennzeichnet, dass**
a) ein Implantatgrundkörper bereitgestellt wird,
b) der Implantatgrundkörper aus Schritt a) so behandelt wird, dass die Oberfläche des Implantates einen Benetzungswinkel von Θ ≤ 80° hat.

Ein dritter Erfindungsgegenstand betrifft eine Verwendung eines erfindungsgemäßen Implantates zur Verringerung der Dosis und/oder der Konzentration der Verabreichung einer systemischen Begleitmedikation mit ein oder mehreren blutgerinnungshemmenden Wirkstoffen vor, während und/oder nach Implantateinsetzung in einen menschlichen oder tierischen Organismus.

Ein vierter Erfindungsgegenstand betrifft ein Verfahren zur Verringerung der Dosis und/oder Dauer der Verabreichung einer systemischen Begleitmedikation mit ein oder mehreren blutgerinnungshemmenden Wirkstoffen vor, während und/oder nach Implantierung eines Implantates in einen menschlichen oder tierischen Organismus, **dadurch gekennzeichnet, dass** ein erfindungsgemäßes Implantat in den menschlichen oder tierischen Organismus implantiert wird.

Bevorzugte Ausgestaltungen der erfindungsgemäßen Gegenstände werden in den abhängigen Ansprüchen sowie der nachfolgenden detaillierten Beschreibung dargestellt und sind, sofern sinnvoll, untereinander kombinierbar.

### Figurenbeschreibung:

Die Figuren zeigen einen schematischen Ausschnitt von erfindungsgemäß zu verwendenden hyperverzweigten Polymeren, insbesondere Sternpolymeren als blutgerinnungshemmende Wirkstoffe für erfindungsgemäße Implantate.

Von den Figuren ist:
Fig. 1: ein schematisierter Ausschnitt eines hyperverzweigten Polymeraufbaus.

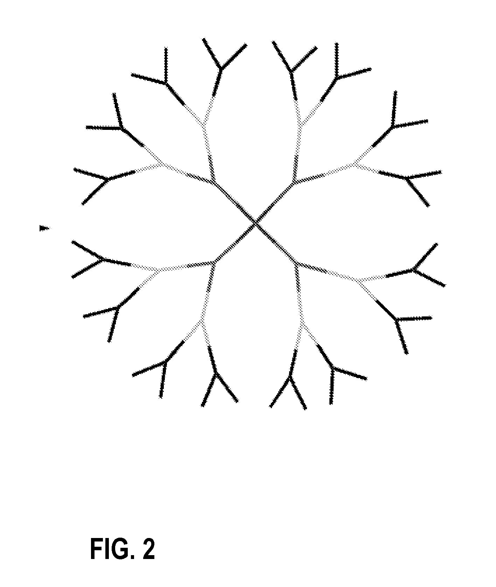

Fig. 2: ein schematisierter Ausschnitt eines Sternpolymer-Aufbaus.

### Detaillierte Beschreibung der Erfindung

Die vorliegende Erfindung beruht auf der Erkenntnis, dass die erfindungsgemäßen Implantate die vorliegende Aufgabe vor allem deshalb lösen, weil die Oberfläche mit einem Benetzungswinkel von Θ ≤ 80° der erfindungsgemäßen Implantate eine verbesserte und insbesondere beschleunigte Endothelialisierung der erfindungsgemäßen Implantate bewirkt.

Aufgrund der verbesserten und insbesondere beschleunigten Endothelialisierung des erfindungsgemäßen Implantates wird die Oberfläche des Implantate beschleunigt mit Endothelzellen bewachsen und somit wird das Anhaften von Thrombozyten und/oder anderen Blutbestandteilen, die eine Thrombose bzw. Thromboembolie verursachen können, verringert oder sogar verhindert. Folglich wird das Thrombose-/Embolierisiko nach Implantation des erfindungsgemäßen Implantates verringert und dadurch kann auch die Dosis und/oder die Konzentration der Verabreichung sowie die Zeitdauer einer systemischen Begleitmedikation mit ein oder mehreren blutgerinnungshemmenden Wirkstoffen verringert werden.

Im Sinne der vorliegenden Erfindung bedeutet Benetzungswinkel von Θ ≤ 80° für die Oberfläche des erfindungsgemäßen Implantates folgendes:

Nach Aufbringen eines Wassertopfens bei Standardbedingungen, nach der Sessile-Drop-Methode, zeigt der Topfen ein von der Oberflächenenergie des Substrates abhängiges Benetzungsverhalten, welches sich im Benetzungswinkel vorn Θ ≤ 80° äußert. Alternativ zur experimentellen Methode kann der Benetzungswinkel gemäß üblicher Methoden berechnet werden. Hierzu kann insbesondere die Du Noüy Ringmethode oder Wilhelmy Plattenmethode herangezogen werden, bei der bei bekannter Oberflächenspannung der Flüssigkeit der Winkel berechnet werden kann.

Im Sinne der vorliegenden Erfindung bedeutet "Behandlung der Oberfläche eines Implantatgrundkörpers damit die Oberfläche einen Benetzungswinkel von Θ ≤ 80° hat" folgendes: Die Oberfläche des Implantats kann üblicherweise durch Auswahl von (i) geeigneten Implantatmaterialien und/oder (ii) geeignete Oberflächenmodifikationen mittels geeigneter hydrophiler Substanzen zur Hydrophilisierung der Oberfläche, und damit zur Einstellung eines Benutzungswinkels Θ ≤ 80° veranlasst werden.

Implantate bzw. Implantatgrundkörper im Sinne der vorliegenden Erfindung können alle medizinischen, plastischen und/oder funktionellen Implantate bzw. Implantatgrundkörper darstellen und werden beispielsweise ausgewählt aus der Gruppe bestehend aus Herzschrittmacher; Hirnschrittmacher; Defibrillatoren; Herzimplantate, insbesondere, aber nicht beschränkt auf Herzklappen; Schrittmacherelektroden, Defibrillationselektroden; Cochleaimplantate; Schwellkörperimplantate; Implantate für die Zahnmedizin; Endoprothesen, bevorzugt für Kniegelenke und Hüftgelenke; Depotimplantate, die dazu dienen, ein Depot eines Wirkstoffs zu bilden; biodegradierbare oder dauerhafte, koronare oder periphere Stents; biodegradierbare oder dauerhafte Stents für andere Hohlräume, vorzugsweise die Speiseröhre, den Gallengang, die Harnröhre, die Prostata oder die Luftröhre; und local drug delivery (LDD)-Implantate, die vorzugsweise endovaskulär im Blut oder anderen Hohlräumen implantiert werden sein.

In einer bevorzugten Ausgestaltung der vorliegenden Erfindung werden erfindungsgemäßen Implantate ausgewählt aus der Gruppe bestehend aus: Herzschrittmacher; Defibrillatoren; Herzimplantate, bevorzugt Herzklappen; Schrittmacherelektroden; Defibrillationselektroden; biodegradierbare oder dauerhafte, koronare oder periphere Stents; und local drug delivery (LDD)- Implantate, die vorzugsweise endovaskulär im Blut oder anderen Hohlräumen implantiert werden sein.

In einer besonders bevorzugten Ausgestaltung der vorliegenden Erfindung werden erfindungsgemäße Implantate ausgewählt aus der Gruppe der dauerhaften oder biodegradierbaren koronaren Stents (z. B. Koronarstent), wobei das Stentgrundkörpermaterial Metall und/oder Polymer umfassen kann.

Die ursprünglichen mechanischen Funktionen eines Koronarstents, beispielsweise die Dilatierbarkeit, der geringe Recoil, die Stabilität über eine gewünschte Zeitdauer (im Falle degradierbarer Stents, z. B. bestehend aus Magnesium und seinen Legierungen) sowie die Flexibilität sollen in erfindungsgemäßen Stents als Implantate bevorzugt vorhanden sein.

Im Folgenden werden erfindungsgemäß zu verwendende Implantat-Materialien, bevorzugt Stentgrundkörper-Materialien sowie bevorzugte Ausgestaltungen hiervon beschrieben:

### Biodegradierbarer Implantatgrundkörper, insbesondere biodegradierbarer Stentgrundkörper:

Im Sinne der vorliegenden Erfindung bedeutet "biodegradierbarer Implantat(grundkörper)", insbesondere "biodegradierbarer Stent(grundkörper)", dass der Grundkörper in physiologischer Umgebung, insbesondere im Gefäßsystem eines menschlichen oder tierischen Körpers degradiert, d. h. so abgebaut wird, dass der Stent seine Integrität verliert. Vorzugsweise degradieren biodegradierbare Implantatgrundkörper erst dann, wenn die Funktion des Implantates nicht mehr physiologisch sinnvoll bzw. notwendig ist. Bei biodegradierbaren Stents bedeutet das, dass der Stent vorzugsweise erst dann degradiert oder seine Integrität verliert, wenn das traumatisierte Gewebe des Gefäßes verheilt ist und der Stent somit nicht länger seine Stützfunktion im Gefäß ausüben muss.

### Metallische Grundkörper:

In einer erfindungsgemäßen Ausgestaltung umfasst oder besteht der biodegradierbare Werkstoff bevorzugt aus einem metallischen Werkstoff, der eine biokorrodierbare Legierung darstellt, wobei die Hauptkomponente der Legierung ausgewählt wird aus der Gruppe Magnesium, Eisen, Zink und Wolfram; insbesondere wird für einen degradierbaren metallischen Werkstoff eine Magnesiumlegierung bevorzugt.

Die Legierung, insbesondere umfassend Magnesium, Eisen, Zink und/oder Wolfram, ist so in ihrer Zusammensetzung zu wählen, dass sie biokorrodierbar ist. Als biokorrodierbar im Sinne der vorliegenden Erfindung werden Legierungen bezeichnet, bei denen in physiologischer Umgebung ein Abbau stattfindet, der letztendlich dazu führt, dass der gesamte Stent oder der aus dem Werkstoff gebildete Teil des Stents seine mechanische Integrität verliert. Unter Legierung wird vorliegend ein metallisches Gefüge verstanden, deren Hauptkomponente Magnesium, Eisen, Zink oder Wolfram ist. Hauptkomponente ist die Legierungskomponente, deren Gewichtsanteil an der Legierung am Höchsten ist. Ein Anteil der Hauptkomponente beträgt vorzugsweise mehr als 50 Gew.%, weiter bevorzugt mehr als 70 Gew.%. Eine Magnesiumlegierung ist bevorzugt.

Ist der Werkstoff eine Magnesiumlegierung, so enthält diese vorzugsweise Yttrium und weitere Seltenerdmetalle, da sich eine derartige Legierung aufgrund ihrer physikochemischen Eigenschaften und ihrer hohen Biokompatibilität, insbesondere auch ihrer Abbauprodukte, auszeichnet.

Besonders bevorzugt werden Magnesiumlegierungen der WE-Reihe, insbesondere WE43 sowie Magnesiumlegierungen der Zusammensetzung Seltenerdmetalle 0,05 - 9,9 Gew.%, davon Yttrium 0,0 - 6,5 Gew.% und einem Rest < 1 Gew.%, der Zirkonium und/oder Silizium enthalten kann, wobei Magnesium den auf 100 Gew.% fehlenden Anteil an der Legierung einnimmt, eingesetzt. Diese Magnesiumlegierungen bestätigten bereits experimentell und in ersten klinischen Versuchen ihre besondere Eignung, d. h. sie zeigen eine hohe Biokompatibilität, günstige Verarbeitungseigenschaften, gute mechanische Kennwerte und ein für die Einsatzzwecke adäquates Korrosionsverhalten. Unter der Sammelbezeichnung "Seltenerdmetalle" werden vorliegend Scandium (21), Yttrium (39), Lanthan (57) und die 14 an Lanthan (57) folgenden Elemente, nämlich Cer (58), Neodym (60), Promethium (61), Samarium (62), Europium (63), Gadolinium (64), Terbium (65), Dysprosium (66), Holmium (67), Erbium (68), Thulium (69), Ytterbium (70) und Lutetium (71), verstanden.

### Polymer-Grundkörper:

Implantatgrundkörper, insbesondere Stentgrundkörper, können gemäß einer weiteren alternativen Ausgestaltung biodegradierbare Polymere umfassen oder daraus bestehen, vorzugsweise ausgewählt aus der Gruppe bestehend aus: Polydioxanon; Polyglycolid; Polycaprolacton; Polyhydroxyvaleriansäure, Polyhydroxybuttersäure, Polylactide, vorzugsweise Poly(L-lactid), Poly(D-lactid), Poly(D,L-lactid) und Blends sowie Copolymere, und vorzugsweise Poly(L-lactid-co-glycolid), Poly(D,L-lactid-co-glycolid), Poly(L-lactid-co-D,L-lactid), Poly(L-lactid-co-trimethylencarbonat), Poly-ε-caprolacton, Poly(L-lactid-co-ε-caprolacton und Tri-Blockcopolymere; Polyesteramid; Polysaccharide, vorzugsweise Chitosan, Alginat, Carragenan, Levan, Hyaluronsäure, Heparin, Dextran und Cellulose oder Cellulosederivaten wie Nitrocellulose; und Polypeptide.

### Dauerhafter Implantatgrundkömer, vorzugsweise dauerhafter Stentgrundkörper:

Im Gegensatz zum biodegradierbaren Grundkörper wird der "dauerhafte Implantatgrundkörper", vorzugsweise der "dauerhafte Stentgrundkörper" in physiologischer Umgebung im menschlichen oder tierischen Körper im Wesentlichen nicht abgebaut, so dass er seine Integrität behält.

### Metallische Grundkörper:

In einer weiteren erfindungsgemäßen Ausgestaltung umfasst oder besteht der Grundkörper eines dauerhaften Implantats, insbesondere eines dauerhaften Stents, vorzugsweise aus einem Formgedächtnis-Material aus einem oder mehreren Materialien ausgewählt aus der Gruppe der Nickel-Titan-Legierungen und Kupfer-Zink-Aluminium-Legierungen, vorzugsweise aus Nitinol.

In einer besonders bevorzugten erfindungsgemäßen Ausgestaltung umfasst oder besteht der Grundkörper eines dauerhaften Implantates, insbesondere eines dauerhaften Stents, aus Edelstahl, vorzugsweise aus einem Cr-Ni-Fe-Stahl - hier bevorzugt die Legierung 316L - oder einem Co-Cr-Stahl.

### Polymer Grundkörper:

In einer weiteren erfindungsgemäßen Ausgestaltung umfasst oder besteht der Grundkörper eines dauerhaften Implantats, insbesondere eines dauerhaften Stents, vorzugsweise aus Polypropylen, Polyethylen, Polyvinylchlorid, Polymethylmethylethylacrylat, Polymethylethylacrylat, Polytetrafluorethylen, Polyvinylalkohl, Polyurethan, Polybuthylen, Terephtalat, Silicon, Polyphosphatcan sowie deren Copolymere und Blends, oder Polyhydroxybuttersäure (ataktisch, isotaktisch, syndiotaktisch sowie deren Blends).

Die Aufgabe der vorliegenden Erfindung wird bevorzugt mit dauerhaften Implantaten, vorzugsweise Stents, insbesondere aus Metall oder biodegradierbaren Implantaten, vorzugsweise Stents, aus Polymer gelöst, da diese dauerhaft oder über eine lange Zeit im Organismus verbleiben und so das Thrombose/Embolierisiko an sich hoch ist.

Im Gegensatz hierzu degradieren metallische biodegradierbare Implantate, vorzugsweise Magnesium-Stents, vergleichsweise schnell, so dass manchmal das Implantat nicht mehr über die gewünschte Dauer seine (Stütz-)funktionalität ausüben kann. Durch die vergleichsweise schnelle Endothelialisierung eines erfindungsgemäßen biodegradierbaren metallischen Implantats, vorzugsweise Stents, wird jedoch die Diffusion von Flüssigkeit, insbesondere Wasser an das Implantatmaterial reduziert. So kann die insbesondere Wasser an das Implantatmaterial reduziert. So kann die Degradierung soweit verzögert werden, dass das Implantat seine (Stütz-) funktionalität über den gesamten gewünschten Zeitraum ausüben kann und gleichzeitig das Thrombo-/Embolierisiko reduziert ist.

In einer weiteren bevorzugten Ausgestaltung kann der Grundkörper des Implantats, vorzugsweise Stents, zusätzlich Kunststoff, vorzugsweise Polyetherurethan, und/oder Keramik und/oder weitere Polymerbeschichtungen umfassen.

Werden im Sinne der vorliegenden Erfindung endovaskulär implantierbare Stents als implantierbare Grundkörper verwendet, so können alle üblichen Stentgeometrien verwendet werden. Insbesondere bevorzugt sind Stentgeometrien, die insbesondere in US 6,896,695, US 2006/241742, US 5,968,083 (Tenax), EP 1 430 854 (Helix-Design), US 6,197,047 und EP 0 884 985 beschrieben werden.

Damit die Oberfläche der erfindungsgemäßen Implantate, vorzugsweise Stents, einen Benetzungswinkel von Θ ≤ 80° aufweist, kann gemäß einer bevorzugten erfindungsgemäßen Ausgestaltung das Implantat- bzw. Stentgrundkörpermaterial ausgewählt werden aus der Gruppe bestehend aus:
- dauerhafte metallischen Materialien: 316 L, Nitinol und Co-Cr, wobei die Materialien allein oder in Kombination mit einer Beschichtung aus Siliziumkarbid (beschichtet nach dem CVD-Verfahren) als Implantatgrundkörper, bevorzugt Stentgrundkörper, vorliegen können,
- dauerhafte Polymermaterialien: Polypropylen, Polyethylen, Polyvinylchlorid, Polymethylmethylethylacrylat, Polymethylethylacrylat, Polytetrafluorethylen, Polyvinylalkohl, Polyurethan, Polybuthylen, Terephtalat, Silicon, Polyphosphatcan sowie deren Copolymere und Blends, oder Polyhydroxybuttersäure (ataktisch, isotaktisch, syndiotaktisch sowie deren Blends),
- biodegradierbare metallische Materialien: Magnesiumlegierungen, insbesondere bevorzugte Magnesiumlegierungen wie vorstehend beschrieben,
- biodegradierbare Polymermaterialien: Polydioxanon; Polyglycolid; Polycaprolacton; Polyhydroxyvaleriansäure, Polyhydroxybuttersäure, Polylactide, vorzugsweise Poly(L-lactid), Poly(D-lactid), Poly(D,L-lactid) und Blends sowie Copolymere, und vorzugsweise Poly(L-lactid-co-glycolid), Poly(D,L-lactid-co-glycolid), Poly(L-lactid-co-D,L-lactid), Poly(L-lactid-co-trimethylencarbonat), Poly-ε-caprolacton, Poly(L-lactid-co-ε-caprolacton und Tri-Blockcopolymere; Polyesteramind, Polysaccharide, vorzugsweise Chitosan, Alginat, Carragenan, Levan, Hyaluronsäure, Heparin, Dextran und Cellulose oder Cellulosederivaten wie Nitrocellulose; und Polypeptide.

Alternativ oder kumulativ zu den vorstehenden Methoden kann die Oberfläche eines erfindungsgemäßen Implantat- bzw. Stentgrundkörpers mit (a) ein oder mehreren, gleichen oder unterschiedlichen hydrophilen Substanzen modifiziert werden, damit die Oberfläche des Implantats einen Benetzungswinkel von Θ ≤ 80° aufweist. Im Sinne der vorliegenden Erfindung bedeutet "modifiziert", dass die Oberfläche der erfindungsgemäßen Implantate, vorzugsweise Stents, so beschichtet ist, dass ein oder mehrere, gleiche oder unterschiedliche, hydrophile Substanzen dauerhaft an die Oberfläche des Implantats bzw. Stents anhaften und nach Implantation nicht an den Organismus abgegeben werden. Übliche Kopplungsmethoden werden in den Ausführungsbeispielen 1 bis 3 dargestellt oder beispielsweise in der folgenden Literaturstelle beschrieben: G.T. Hermanson; Bioconjugate Techniques: 1996, Academic Press, ISBN 0-12-342336-8.

In einer bevorzugten Ausgestaltung werden die (a) hydrophilen Substanzen ausgewählt aus der Gruppe von Hyaluronsäure, bevorzugt vernetzte oder derivatisierte Hyaluronsäure; Chondroitinsulfat; extrazellulare Matixpoly- oder Oligopeptide der SEQ ID Nr. 1 oder der SEQ ID Nr. 2 und Fragmenten oder Derivaten hiervon.

In einer weiteren bevorzugten erfindungsgemäßen Ausgestaltung wird die Oberfläche eines erfindungsgemäßen Implantats, vorzugsweise Stents zusätzlich mit (b) ein, zwei oder mehreren, gleichen oder unterschiedlichen, blutgerinnungshemmenden Wirkstoffen beschichtet.

Ein Wirkstoff im Sinne dieser Erfindung ist eine Substanz oder eine Verbindung, die im menschlichen oder tierischen Körper eine biologische Reaktion hervorruft. Ein blutgerinnungshemmender Wirkstoff ruft dementsprechend in einem menschlichen oder tierischen Körper eine blutgerinnungshemmende Reaktion hervor. In diesem Sinne kann Wirkstoff auch synonym zu Arzneistoff und/oder Pharmakon verwendet werden.

In einer bevorzugten Ausgestaltung werden (b) ein, zwei oder mehrere, gleiche oder unterschiedliche, blutgerinnungshemmende Wirkstoffe dauerhaft an die Oberfläche des Implantats bzw. Stents gebunden, so dass diese nicht nach Implantation an den Organismus abgegeben werden. Ein oder mehrere blutgerinnungshemmende Wirkstoffe, insbesondere Peptide der SEQ ID Nr. 3 und SEQ ID Nr. 4, können ebenfalls hydrophile Eigenschaften aufweisen und so den Einstellung des Benetzungswinkels von Θ ≤ 80° und damit die verbesserte und insbesondere beschleunigte Endothelialisierung der erfindungsgemäßen Implantate zusätzlich fördern. Zudem kann die Endothelialisierung dadurch zusätzlich unterstützt werden, dass das Anhaften von Thrombozyten und/oder anderen Blutbestandteilen, die eine Thrombose bzw. Embolie verursachen können, an die Oberfläche des erfindungsgemäßen Implantates direkt durch die blutgerinnungshemmend wirkenden Wirkstoffe verringert oder sogar verhindert wird. Folglich sind die Implantate, vorzugsweise Stents, die zusätzlich (b) ein oder mehrere blutgerinnungshemmende Wirkstoffe aufweisen bevorzugt, da diese zu einer Verringerung der Dosis und/oder Konzentration der Verabreichung einer systemischen Begleitmedikation mit ein oder mehreren blutgerinnungshemmenden Wirkstoffen beitragen.

In einer besonders bevorzugten Ausgestaltung werden (b) der oder die blutgerinnungshemmenden Wirkstoffe ausgewählt aus der Gruppe bestehend aus: blutgerinnungshemmenden Peptide, bevorzugt Peptide der SEQ ID Nr. 3 oder der SEQ ID Nr. 4 oder Fragmente oder Derivate hiervon; Glucosaminglykane, bevorzugt Heparin; Vitamin-K-Antagonisten, bevorzugt Cumarin, Dicoumarol, Phenprocoumon, Warfarin und Acenocoumarol; sulfatierte blutgerinnungshemmende Polymere, bevorzugt sulfatierte hyperverzweigte Polymere, sulfatierte Sternpolymere; und Dendrimere, bevorzugt sulfatierte Dendrimere.

In einer besonders bevorzugten Ausgestaltung werden (b) der oder die blutgerinnungshemmenden Wirkstoffe ausgewählt aus der Gruppe bestehend aus: Peptide der SEQ ID Nr. 3 oder der SEQ ID Nr. 4 oder Fragmenten oder Derivaten hiervon; Cumarin, Phenprocoumon, Warfarin und Acenocoumarol; sulfatierte Sternpolymere; sulfatierte hyperverzweigte Polymere; Dendrimere und sulfatierte Dendrimere.

Im Sinne der vorliegenden Erfindung umfasst der Begriff "hyperverzweigte Polymere" alle Makromoleküle, die in regelmäßiger oder unregelmäßiger Form starke Verzweigungen aufweisen.

Im Sinne der vorliegenden Erfindung bedeutet der Begriff "Sternpolymer", dass das Polymer eine Untereinheit der hyperverzweigten Polymere bildet, bei denen von einem Zentrum 3 oder mehr Ketten ausgehen. Das Zentrum kann dabei ein einzelnes Atom (Beispielsweise Stickstoff) oder eine Atomgruppe (beispielsweise eine organische Kohlenwasserstoffverbindung insbesondere in Ringform) sein. Sternpolymere können entweder Arme gleicher Länge und Zusammensetzungen enthalten oder asymmetrisch aufgebaut sein, d. h. unterschiedliche Armlänge und Blockcopolymer-Ketten.

Im Sinne der vorliegenden Erfindung bedeutet "Dendrimer" eine besondere Untereinheit der Sternpolyere, wobei in den Armen weitere Verzweigungen auftreten.

Während Dendrimere schrittweise aufgebaut werden, erfolgt die Synthese der "einfacheren" hochverzweigten Strukturen in einem Ansatz durch den Umsatz eines Monomers der Struktur ABₙ, das eine reaktive A-Gruppe und n reaktive B-Gruppen enthält. Durch die Reaktion der A-Gruppen mit den B-Gruppen entstehen statistisch verzweigte Moleküle. Es kommt dabei nicht zu Vernetzungsreaktionen, da die B-Gruppen im Überschuss vorliegen und zu wenige "Partner" zur Ausbildung von Netzstrukturen finden.

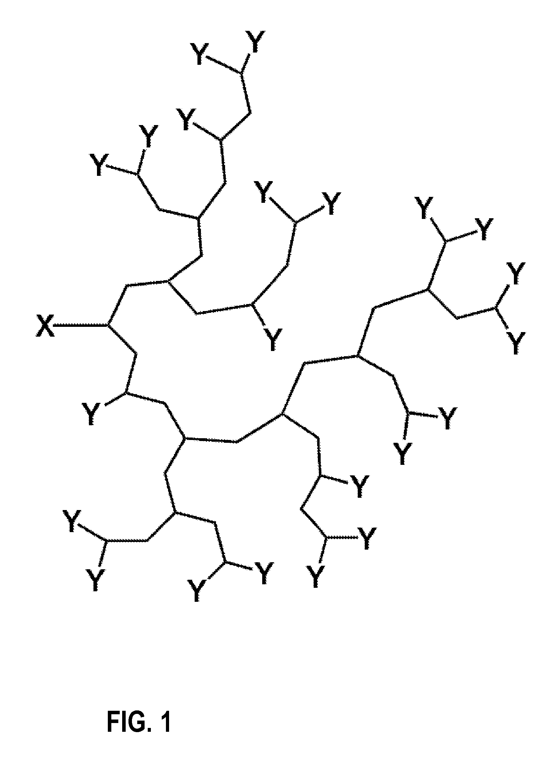

In den folgenden Literaturstellen werden Herstellungsverfahren von hyperverzweigten Polymeren (siehe auch Figur 1; 4 = -SO₃), bevorzugt Sternpolymeren beschrieben: Zilliox J. G., Rempp P., Parrod J. : Pol. Sci. C; 22, 145, (1966) und Rempp P., Franta E.: Pure and Appl. Chem.; 30, 229, (1972). Demnach wird die Herstellung eines Sternpolymers aus Styrol und Divinylbenzol in der Art beschrieben, dass das Divinylsulfon erst zugesetzt wird, nachdem der Styrol-Zulauf beendet ist. Dadurch entstehen Polymere mit Doppelbindungen am Kettenende bei gleichzeitigem einseitigem Wachstum und erfindungsgemäße zu verwendende Sternpolymere bilden sich durch Reaktion an der nicht wachsenden Kette.

Eine alternative Herstellungsmethode von hyperverzweigten Polymeren, bevorzugt Sternpolymeren, kann mittels anionischer Polymerisation durchgeführt werden und wird in der folgenden Literaturstelle beschrieben: Nagasawa M., Fujimoto T.: Progr. Pol. Sci. Japan; 2, 263, (1972). Hierbei wird als Initiator ein polyfunktionelles Anion so eingesetzt, dass ein Makromolekül sternförmig nach allen Seiten wächst. Polyfunktionelle Initiatoren mit mehreren anionischen Resten erhält man durch Polymerisation von Divinylbenzol mit Butyl-Litium in verdünnter Lösung (Eschwey H., Hallensleben M.L., Burchard W.: Makro. Ch.; 173, 235-239, (1973)).

In der folgenden Literaturstelle wird eine Sulfatierungsmethode mittels eines SO₃-Pyridinkomplexes für hyperverzweigte Polymeren, bevorzugt Sternpolymere und Dendrimere beschrieben (Sunder A., Hanselmann R., Frey H., Müllhaupt R.: Macromolecules; 32, (1999) Sunder A., Mühlhaupt, R., Haag R., Frey, H.: Macromolecules, 33, 253, (2000)).

Üblicherweise können die (b) ein oder mehreren, gleichen oder unterschiedlichen blutgerinnungshemmenden Wirkstoffe an funktionalisierte Oberflächen erfindungsgemäßer Implantate gebunden werden. Die Oberflächen können beispielsweise dopaminisiert oder silanisiert (Ausführungsbeispiel 3) sein. Nicht limitierende Beispiele hierzu werden in Ausführungsbeispielen 4 und 5 dargestellt.

Blutgerinnungshemmende Peptide können ebenfalls mittels üblicher Kopplungsreaktionen, wie sie auch zur Immobilisierung von Enzymen verwendet werden, an die Oberfläche der Implantate, vorzugsweise Stents, gebunden werden. Hierzu zählen Verfahren der ionotropen Gelbildung, beispielsweise mittels Alginat oder Chitosan, und insbesondere der Symplexgelbildung, beispielsweise mittels Alginat-Chitosan. Geeignete Verfahren werden insbesondere in der Dissertationsschrift von Alexander Borck, "Herstellung und Untersuchung von biokompatiblen Materialien für medizintechnische Anwendungen", Universität Braunschweig; URL: http://www.digibib.tu-bs.de/?docid=00000014; Kapitel 2.1.1 mit weiteren Nachweise, beschrieben.

Sulfatierte Polymere, vorzugsweise sulfatierte hyperverzweigte Polymere, weiter bevorzugt sulfatierte Sternpolymere, sowie sulfatierte Dendrimere können üblicherweise mittels kovalenter Bindungen oder mittels ionischer Wechselwirkungen, insbesondere ionotroper Gelbildung mit kationischen Polyelektrolyten, beispielsweise Chitosan, Poly-diallyldimethyl-ammoniumchlorid (Poly-DADMAC) und Polyethylenimin, in Form von Symplexgelen, beispielsweise Alginat/Chitosan, als Monolagen auf die Oberfläche der erfindungsgemäßen Implantate, vorzugsweise Stents, gebunden werden (siehe hierzu Dissertationsschrift von Alexander Borck, "Herstellung und Untersuchung von biokompatiblen Materialien für medizintechnische Anwendungen", Universität Braunschweig; URL: http://www.digibib.tu-bs.de/?docid=00000014; Kapitel 2.1.1.3; 3.2.1.1.4 und 4.1.2 mit weiteren Nachweisen).

Alginat wird üblicherweise durch mehrvalente Kationen, Ca²⁺ oder Al³⁺, in den wasserunlöslichen Zustand überführt, während beim Chitosan ein mehrwertiges Phosphat eingesetzt wird. Möglich ist aber auch ein Symplexgel, bei dem das Polykation Chitosan mit dem Polyanion Alginat wechselwirkt. Die Symplexgel-Bildung geschieht über eine Metathese, eine doppelte Umsetzung.

Das Chitosan reagiert mit dem Polyphosphat und führt zu einer Strukturgebung. Das Ca-Alginat reagiert mit dem Polyphosphat unter Ausbildung des schlecht löslichen Ca-Polyphosphats und des löslichen Na-Alginats, das seinerseits in Konkurrenz mit dem chitosangebundenen Polyphosphat tritt. Es bildet sich Alginat/Chitosan, ein Symplexgel, dass dann als Monolage auf eine Stentoberfläche beschichtet werden kann. Ein nicht limitierendes Beispiel hierzu wird in Ausführungsbeispiel 4 dargestellt.

In einer weiteren bevorzugten Ausgestaltung umfasst das erfindungsgemäße Implantat, vorzugsweise Stent, eine Beschichtung mit einer effektiven Konzentration an (c) ein oder mehreren, gleichen oder unterschiedlichen weiteren Wirkstoffen, um Spätkomplikationen, wie "In-Stent-Restenose", Gewebeinflammationen oder andere Erkrankungen, wie beispielsweise Krebserkrankungen, zu behandeln. Im Sinne der vorliegenden Erfindung werden (c) der oder die weiteren Wirkstoffe nicht dauerhaft an das Implantat, vorzugsweise Stent gebunden, sondern werden nach Implantation des Implantats, vorzugsweise Stents an den Blutkreislauf und/oder das Gewebe des menschlichen oder tierischen Organismus abgegeben.

Hierfür werden (c) der oder die weiteren Wirkstoffe bevorzugt ausgewählt aus der Gruppe bestehend aus: Antiphlogistika, vorzugsweise Dexamethason, Methylprednisolon und Diclophenac; Cytostatika, Taxole vorzugsweise Paclitaxel, Colchicin, Actinomycin D und Methotrexat; Immunsuppressiva, vorzugsweise Limus-Verbindungen, weiter bevorzugt Sirolimus (Rapamycin) und seine Derivate, Zotarolimus (Abt-578), Tacrolimus (FK-506), Everolimus, Biolimus, insbesondere Biolimus A9 und Pimecrolimus, Cyclosporin A und Mycophenolsäure; Thrombozytenaggregationshemmer, vorzugsweise Abciximab und Iloprost; Statine, vorzugsweise Simvastatin, Mevastatin, Atorvastatin, Lovastatin, Pitavastatin und Fluvastatin; und Estrogene, vorzugsweise 17b-Estradiol, Daizein und Genistein; Lipidregulatoren, vorzugsweise Fibrate; Immunsuppressiva; Vasodilatatoren, vorzugsweise Satane; Calciumkanalblocker; Calcineurininhibitoren, vorzugsweise Tacrolimus; Antiinflammatorika, vorzugsweise Imidazole; Antiallergika; Oligonucleotide, vorzugsweise Decoy-Oligodesoxynukleotid (dODN); Endothelbildner, vorzugsweise Fibrin; Steroide; Proteine/Peptide; Proliferationshemmer; Analgetika und Antirheumatika.

Besonders bevorzugt werden erfindungsgemäß Paclitaxel und Limus-Verbindungen, weiter bevorzugt Sirolimus (Rapamycin), Zotarolimus (Abt-578), Tacrolimus (FK-506), Everolimus, Biolimus, insbesondere Biolimus A9 und Pimecrolimus, ganz besonders bevorzugt Rapamycin (Sirolimus) als (c) der oder die weiteren Wirkstoffe verwendet.

Ein erfindungsgemäßer peripherer oder koronarer Stent wird vorzugsweise auf der abluminalen Seite, also der Oberfläche, die mit dem Gewebe und nicht dem Gefäßlumen des Blutgefäßes nach Implantation in Kontakt steht, mit (c) dem oder den weiteren Wirkstoffen beschichtet, da bei einer zusätzlichen luminalen Beschichtung die Degradation des Stents, vorzugsweise eines biodegradierbaren Stents und besonders bevorzugt eines biodegradierbaren Metallstents wesentlich beeinträchtigt wird.

In einer weiteren bevorzugten Ausgestaltung kann ein mit weiteren Wirkstoffen beschichtetes erfindungsgemäßes Implantat zusätzlich (d) eine weitere (wirkstofffreie) Beschichtung als sogenannten "Topcoat" aufweisen, um die Abrasion der Wirkstoffbeschichtung bei Implantation zu verringern.

Die Beschichtung der Oberfläche des erfindungsgemäßen Implantats, vorzugsweise Stents, mit (c) weiteren Wirkstoffen wird gemäß üblicher Verfahren bewerkstelligt. Insbesondere kann hierzu eine reine Wirkstoffschmelze, ein Wirkstoff-Lösungsmittelgemisch oder ein Wirkstoff-Polymergemisch beispielsweise mittels eines Tauchverfahren (Dippcoating), einer Sprühbeschichtung mittels Ein- bzw. Mehrstoffdüse, Rotationszerstäubung und Druckdüsen, Sputtern auf die Oberfläche des Implantats aufgetragen werden. Gleiche Beschichtungsverfahren können auch bevorzugt für den (d) Topcoat angewendet werden.

Für den Fall, dass ein oder mehrere unterschiedliche Polymere für die (c) weitere Wirkstoffbeschichtung und/oder den (d) Topcoat verwendet werden, werden sie üblicherweise ausgewählt aus der Gruppe bestehend aus:
- nicht degradierbare Polymere: Polyethylen; Polyvinylchlorid; Polyacrylate; vorzugsweise Polyetyl- und Polymethylacrylate, Polymethylmetacrylat, Polymethyl-coethyl-acrylat und Ethylen/Ethylacrylat; Polytetrafluorethylen, vorzugsweise Ethylen/Chlortrifluorethylen Copolymere, Ethylen/Tretrafluorethylen Copolymere; Polyamide, vorzugsweise Polyamidimid, PA-11, PA-12, PA-46, PA-66; Polyetherimid; Polyethersulfon; Poly(iso)butylen; Polyvinylchlorid; Polyvinylfluorid; Polyvinylalkohol; Polyurethan; Polybuthylenterephthalat; Silikone; Polyphosphazen; Polymerschäume, vorzugsweise Polymerschäume aus Carbonaten, Styrolen; Copolymere und/oder Blends der aufgezählten Polymerklassen, Polymere der Klasse der Thermoplaste sowie
- degradierbare Polymere: Polydioxanon; Polyglycolid; Polycaprolacton; Polylactide, vorzugsweise Poly-L-Lactid, Poly D,L Lactid, und Copolymere sowie Blends hiervon, vorzugsweise Poly(L-Lactid-co-glycolid), Poly(D,L-lactid-co-glycolid), Poly(L-Lactid-co-D,L-Lactid), Poly(1-Lactid-co-trimethylen carbonat); Triblockcopolymere; Polysaccharide, vorzugsweise Chitosan, Levan, Hyaluronsäure, Heparin, Dextran, Cellulose; Polyhydroxyvalerat; Ethylvinylacetat; Polyethylenoxid; Polyphosphorylcholin; Fibrin; Albumin; Polyhydroxybuttersäure, vorzugsweise ataktische, isotaktische und/oder syndiotaktische Polyhydroxybuttersäure sowie deren Blends.

Besonders bevorzugte Polymere für die (c) wirkstoffhaltige Schicht oder den (d) Topcoat der vorliegenden Erfindung sind die oben beschriebenen degradierbaren Polymere, da durch den vollständigen Abbau des oder der Polymere kein körperfremder Bestandteil im Organismus verbleibt.

Für den Fall, dass vorwiegend nur die abluminale Oberfläche eines erfindungsgemäßen Stents mit (c) ein oder mehreren weiteren Wirkstoffen beschichtet werden soll, kann dies vorzugsweise dadurch bewerkstelligt werden, dass in den vorgenannten Verfahren der Stent auf beispielsweise einen Zylinder, Kanüle oder Dorn aufgesteckt wird, damit nur die abluminale Oberfläche des Stents mit einer Wirkstoffschicht beschichtet wird. Alternativ könnte die abluminale Beschichtung mit weiteren Wirkstoffen über einen Walzenauftrag oder das selektive Auftrag durch Bestreichen, Befüllen von Kavitäten vorgenommen werden. Gleiche Verfahren können auch bevorzugt für den (d) Topcoat angewendet werden.

Gegebenenfalls kann einem oder mehreren Beschichtungsschritten ein üblicher Trocknungsschritt oder andere übliche physikalische oder chemische Nachbearbeitungsschritte, z. B. Vakuum- oder Plasmabehandlung, folgen, bevor das erfindungsgemäße Implantat, vorzugsweise Stent, weiter behandelt wird.

Die bevorzugten Ausgestaltungen des erfindungsgemäß verwendbaren Implantats, vorzugsweise des erfindungsgemäßen Stents können in allen denkbaren Varianten untereinander, aber auch mit den weiteren hierin offenbarten bevorzugten Ausgestaltungen kombiniert werden.

### Ausführungsbeispiele:

Als Stentgrundkörper wird der PRO-Kinetic, ein Cobalt-Chrom-Stent mit ProBio-Beschichtung, bestehend aus einer Siliziumkarbid-Schicht verwendet.

Die vorliegende Erfindung wird im Folgenden durch Ausführungsbeispiele beschrieben, die jedoch den Schutzumfang des erfindungsgemäßen Gegenstandes nicht limitieren.

### Ausführungsbeispiel 1: Kopplung mit Carbonyldimidazol (CDI):

Ein im Sauerstoffplasma oder durch Spülen mit der Lösungsmittelreihe Dichlormethan, Aceton, Methanol und Millipore Wasser gereinigter Stent wird im Folgenden weiterbehandelt:

Es wird eine 1 mM Lösung von Hydroxyundecylphosphonsäure in trockenem Tetrahydrofuran hergestellt. In diese wird der Stent gehängt und das Lösungsmittel wird innerhalb von einer Stunde abgedampft, wobei der Meniskus der Lösung über die Stentoberfläche wandert.

Anschließend wird der Stent 18 Stunden bei 120°C getempert und daraufhin mit Lösungsmittel gespült.

Der so vorbehandelte Stent wird 15 Stunden in eine 0,3 M Lösung von Carbonyldiimidazol (CDI) in trockenem Dioxan gelegt. Anschließend wird der Stent zwei mal 10 Minuten mit trockenem Dioxan gespült und anschließend im Stickstoffstrom getrocknet.

Auf die so behandelten Stents wird eine Lösung zu koppelnden Reagenzien, wie den oben beschriebenen Peptiden (ca. 50 µg/ml) in PBS-Puffer (aminosäurefrei) gegeben und über Nacht bei 4°C geschüttelt. Anschließend wurden die Stents mit Puffer gespült.

### Ausführungsbeispiel 2: Kopplung mit 3-(4-Oxybenzophenon)propylphosphonsäure:

Ein gemäß Beispiel 1 gereinigter Stent wird im Folgenden weiterbehandelt:

Es wird eine 3 mM Lösung von 3-(4-Oxybenzophenone)propylphosphonsäure in trockenem Tetrahydrofuran hergestellt.

Mit dieser Lösung wird der gereinigte Stent dreimal besprüht. Anschließend wird der Stent 12 Stunden bei 120°C getempert und daraufhin mit Lösungsmittel gespült.

Diese Stents werden in eine Lösung zu koppelnden Reagenzien, wie den oben beschriebenen Peptiden (ca.500 µg/ml) in Puffer gegeben und über Nacht bei 4°C geschüttelt.

Am nächsten Tag werden die Stents aus dem Lösungsmittel entfernt, getrocknet und bei 260 nm mit 100 mW/cm² belichtet.

Nicht gebundenes Protein wird abgewaschen.

### Ausführungsbeispiel 3: Kopplung mit Silan:

### Ansatz:

Die gereinigten Stents gemäß Beispiel 1 werden in eine Mischung aus Toluol, Triethylamin und 3 Aminopropyltriethoxysilan gegeben. Und 14 Std. bei Raumtemperatur inkubiert. Nach Ablauf der Reaktion wird der Stent in Toluol gewaschen und für 1 Std. bei 135°C getempert.

### Herstellung der Silanisierungslösung:

10 ml Toluol, getrocknet
0,5 ml Trietylamin
1 ml Silan 3 Aminopropyltriethoxysilan

An den Reinigungsschritt (spülen der Stents mit Trichlormethan) schließt sich die Aktivierung mit 1,1'-Carbonyl Diimidazol (CDI) an. Die Qualität des CDI ist entscheidend für das Gelingen.

Die silanisierten und gespülten Stents werden für 5 Stunden in CDI gegeben. Dazu wird das CDI in trocknendem Dioxan gelöst. Hier eignet sich eine Stammlösung von 2,5 g / 50 ml CDI in Dioxan die für mehrere Tage (2, trocken) haltbar ist. Die Stents werden bei Raumtemperatur leicht bewegt.

Nach der Aktivierung werden die Stents entnommen und mit trocknendem Dioxan gespült.

Für die Ankopplung der Peptide werden die aktivierten Stents in die Peptid Lösung getaucht und bei 4°C über Nacht (min. 12 Std.) gekoppelt.
Die Reaktion geschieht am geeignetsten in 125 mM Natriumborat mit 0,066% SDS bei einem pH-Wert von 10.0.
Die Lösung ist danach wiederverwendbar bzw. es können mehrere Oberflächen mit dieser Lösung behandelt werden.

Die Stents werden nach der Kopplung dreimal mit 5 ml des Borax-Puffers (oben) gewaschen. Danach noch dreimal mit Wasser. Die nach diesen Waschschritten noch analysierbaren Peptide sind kovalent gebunden.

### Ausführungsbeispiel 4: Bindung von blutgerinnungshemmenden Wirkstoffen an dopaminisierte Implantatoberflächen am Beispiel von Stentoberflächen

### 4.1 Dopaminisierung einer Stentoberfläche

Die Stentoberfläche wird bei 20°C über 2-6 Stunden mit einer 1-3 %igen L-Dopamin-Lösung in einer 50 mM Phosphatpuffer-Lösung (ohne NaCl Zusatz) in Kontakt gebracht.

### 4.2 Anbindung eines blutgerinnungshemmenden Wirkstoffes an die dopaminisierte Stentoberfläche

Die dopaminisierten Stents werden für 5 Stunden in CDI gegeben. Dazu wird das CDI in trocknendem Dioxan gelöst. Hier eignet sich eine Stammlösung von 2,5 g / 50 ml CDI in Dioxan. Die Stents werden bei Raumtemperatur leicht bewegt.

Nach der Aktivierung werden die Stents entnommen und mit trocknendem Dioxan gespült.

Für die Ankopplung der blutgerinnungshemmenden Peptide oder der beschriebenden Polymer werden die aktivierten Stents in die entsprechenden Lösung getaucht und bei 4°C über Nacht (min. 12 Std.) gekoppelt.

Die Reaktion geschieht am geeignetsten in 125 mM Natriumborat mit 0,066% SDS bei einem pH-Wert von 10.0.

Die Lösung ist danach wiederverwendbar bzw. es können mehrere Oberflächen mit dieser Lösung behandelt werden.

Die Stents werden nach der Kopplung 3 mal mit 5 ml des Borax-Puffers (oben) gewaschen. Danach noch dreimal mit Wasser.

### Ausführungsbeispiel 5: Bindung von blutgerinnungshemmenden Wirkstoffen an silanisierte Implantatoberflächen am Beispiel von Stentoberflächen

### 5.1 Silanisierung einer Stentoberfläche

Analog zu Ausführungsbeispiel 1 kann die Stentoberfläche durch eine Vielzahl von Aminosilanen, beispielsweise 3-Aminopropantrimethoxysilan oder 3-Aminopropantriethoxysilan in Toluol funktionalisiert werden.

Gegebenenfalls müssen die Stentoberflächen, insbesondere bei Stents mit Kunststoff- oder Siliziumcarbidoberflächen beispielsweise mittels üblicher plasmatechnischer Verfahren so vorbehandelt werden, so dass Hydroxy-Gruppen auf der Oberfläche entstehen, die dann in einem weiteren Schritt mit Ethoxy- oder Methoxysilanen gekoppelt werden können. Geeignete Vorbehandlungsverfahren werden beispielsweise in der Dissertationsschrift von Alexander Borck beschrieben: "Herstellung und Untersuchung von biokompatiblen Materialien für medizintechnische Anwendungen", Universität Braunschweig; URL: http://www.digibib.tu-bs.de/?docid=00000014; Kapitel 2.3.2; 3.2.2.

Für die Silanisierung werden 100 µl Triethoxypropylaminosilan in 15 ml trockenem Toluol gelöst. Die Stents werden in trockene Reagenzgläser überführt und mit 2 ml der Silan-Lösung überschichtet. Nach 15 Minuten werden die Stents mit Dichlormethan gespült und bei 75°C für 1 Stunde inkubiert.

### 5.2 Anbindung eines blutgerinnungshemmenden Wirkstoffes an die silanisierte Stentoberfläche

Die Anbindung der Substanzen an die durch Silanisierung aminfunkionalisierten Stents geschieht analog zu der Anbindung der dopaminisierten Methode.

### Ausführungsbeispiel 6: Bindung von blutgerinnungshemmenden Wirkstoffen an mit 1,1'-Carbonyl Diimidazol (CDI) funktionalisierte Implantatoberflächen am Beispiel von Stentoberflächen

### 6.1 1,1'-Carbonyl Diimidazol (CDI) Funktionalisierung einer Stentoberfläche

Eine Stentoberfläche wird bei 20°C über 5 Stunden mit einer Lösung aus 2,5 g 1,1'-Carbonyl Diimidazol (CDI) in 50 ml Dioxan (wasserfrei) in Kontakt gebracht.

### 6.2 Anbindung eines blutgerinnungshemmenden Wirkstoffes an die 1'-Carbonyl Diimidazol (CDI) funktionalisierte Stentoberfläche

Die Ankoppelung ein oder mehrerer blutgerinnungshemmender Peptide wird in einer 125 mM Natriumborat Lösung mit 0,066 % Natrium Dodecylsulfat (sodium dodecyl sulfat, SDS) bei einem pH-Wert 10 durchgeführt. Die Peptidkonzentration in dieser Lösung beträgt 0,01-1 g Peptid in 1 ml Lösung. Der Stent wird mit der Peptid-Lösung bei 5°C über 12 Stunden getaucht.

### Ausführungsbeispiel 7: Symplexgel-Bildung auf Implantatoberflächen am Beispiel von Stentoberfläche

4 ml einer Ca-Alginat Suspension (10 %) in CaCl2 (1 %) werden zu 26 ml Chitosanlösung (Low-viscosity; 25 %; Fa. Fluka) gegeben und suspendiert. Für die Herstellung der Na-Tripolyphoshat-Lösung werden 15 g Tripolyphosphat (Natriumpentaphosphat; Fa. Fluka) in 1 I Wasser bidest. gelöst. Mit 1n HCl wird der pH-Wert auf 6 eingestellt. Der pH-Wert der Alginat/Chitosan-Suspension wird mit 1 n HCl auf 5,5 eingestellt.

Nach Säurezugabe wird die Lösung stark viskos. Diese Lösung wird in 1,5 %ige Penta-Natriumpolyphosphatlösung mit pH-Wert 6 getropft, bzw. ein mit Suspension beschichteter Träger wird in eine 1,5 %ige Penta-Natriumpolyphosphatlösung mit pH-Wert 6 eingetaucht. Nach 50 Minuten können die erfindungsgemäßen Stents aus der Lösung herausgenommen werden.

## Patentansprüche

1. Implantat für einen menschlichen oder tierischen Organismus, **dadurch gekennzeichnet, dass** die Oberfläche des Implantates einen Benetzungswinkel von Θ ≤ 80° hat.

2. Implantat gemäß Anspruch 1, **dadurch gekennzeichnet, dass** das Implantat ausgewählt wird aus der Gruppe bestehend aus: Herzschrittmacher; Hirnschrittmacher; Herzimplantate; Schrittmacherelektroden, Defibrillationselektroden; Cochleaimplantate; Implantate für die Zahnmedizin; Endoprothesen; Depotimplantate, die dazu dienen, ein Depot eines Wirkstoffs zu bilden; biodegradierbare oder dauerhafte, koronare oder periphere Stents; biodegradierbare oder dauerhafte Stents für andere Hohlräume; und local drug delivery (LDD)- Implantate.

3. Implantat gemäß Anspruch 1 oder 2, **dadurch gekennzeichnet, dass** das Implantat ein biodegradierbarer oder ein dauerhafter, koronarer oder peripherer Stent ist.

4. Implantat gemäß nach Anspruch 3, **dadurch gekennzeichnet, dass** der Stentgrundkörper Metall und/oder Polymer umfasst.

5. Implantat gemäß einem der vorstehenden Ansprüche, **dadurch gekennzeichnet, dass** das Implantatmaterial ausgewählt wird aus der folgenden Gruppe, so dass die Oberfläche des Implantats einen Benetzungswinkel von Θ ≤ 80° hat:
Dauerhafte metallische Materialien: 316 L, Nitinol und Co-Cr, wobei die Materialien allein oder in Kombination mit einer Beschichtung aus Siliziumcarbid (beschichtet nach dem CVD-Verfahren) als Implantatgrundkörper, bevorzugt Stentgrundkörper, vorliegen können,
Dauerhafte Polymergrundkörper: Polypropylen, Polyethylen, Polyvinylchlorid, Polymethylmethylethylacrylat, Polymethylethylacrylat, Polytetrafluorethylen, Polyvinylalkohl, Polyurethan, Polybuthylen, Terephtalat, Silicon, Polyphosphatcan sowie deren Copolymere und Blends, oder Polyhydroxybuttersäure (ataktisch, isotaktisch, syndiotaktisch sowie deren Blends),
Biodegradierbare metallische Materialien: Magnesiumlegierungen, insbesondere bevorzugte Magnesiumlegierungen wie vorstehend beschrieben,
Biodegradierbare Polymermaterialien: Polydioxanon; Polyglycolid; Polycaprolacton; Polyhydroxyvaleriansäure, Polyhydroxybuttersäure, Polylactide, vorzugsweise Poly(L-lactid), Poly(D-lactid), Poly(D,L-lactid) und Blends sowie Copolymere, und vorzugsweise Poly(L-lactid-co-glycolid), Poly(D,L-lactid-co-glycolid), Poly(L-lactid-co-D,L-lactid), Poly(L-lactid-co-trimethylencarbonat), Poly-ε-caprolacton, Poly(L-lactid-co-ε-caprolacton und Tri-Blockcopolymere; Polyesteramid, Polysaccharide, vorzugsweise Chitosan, Alginat, Carragenan, Levan, Hyaluronsäure, Heparin, Dextran und Cellulose oder Cellulosederivaten wie Nitrocellulose, Polypeptide.

6. Implantat gemäß einem der vorstehenden Ansprüche, **dadurch gekennzeichnet, dass** die Oberfläche mit (a) ein oder mehreren, gleichen oder unterschiedlichen hydrophilen Substanzen modifiziert wird, damit die Oberfläche des Implantats einen Benetzungswinkel von Θ ≤ 80° hat.

7. Implantat gemäß Anspruch 6, **dadurch gekennzeichnet, dass** die hydrophilen Substanzen ausgewählt werden aus der Gruppe von Hyaluronsäure, bevorzugt vernetzte oder derivatisierte Hyaluronsäure; Chondroitinsulfat, Poly- oder Oligopeptide der SEQ ID Nr. 1 oder der SEQ ID Nr. 2 und Fragmenten oder Derivaten hiervon.

8. Implantat gemäß einem der vorstehenden Ansprüche, **dadurch gekennzeichnet, dass** die Oberfläche zusätzlich mit (b) ein, zwei oder mehreren blutgerinnungshemmenden Wirkstoffen modifiziert ist.

9. Implantat gemäß Anspruch 8, **dadurch gekennzeichnet, dass** der oder die blutgerinnungshemmenden Wirkstoffe ausgewählt werden aus der Gruppe bestehend aus: blutgerinnungshemmende Peptide; Glucosaminglykane; Vitamin-K-Antagonisten; sulfatierte blutgerinnungshemmende Polymere und Dendrimere.

10. Implantat gemäß Anspruch 9, **dadurch gekennzeichnet, dass** der oder die blutgerinnungshemmenden Wirkstoffe ausgewählt werden aus der Gruppe bestehend aus: Peptide der SEQ ID Nr. ..., Cumarin, Phenprocoumon, Warfarin und Acenocoumarol; sulfatierte hyperverzweigte Polymere; sulfatierte Sternpolymere; Dendrimere und sulfatierte Dendrimere.

11. Implantat gemäß einem der vorstehenden Ansprüche, **dadurch gekennzeichnet, dass** Anspruch die Oberfläche zusätzlich mit (c) ein, zwei oder mehreren weiteren Wirkstoffen beschichtet ist.

12. Verfahren zur Herstellung eines Implantates, **dadurch gekennzeichnet, dass**
a) ein Implantatgrundkörper bereitgestellt wird,
b) der Implantatgrundkörper aus Schritt a) so behandelt wird, dass die Oberfläche des Implantates einen Benetzungswinkel von Θ ≤ 80° hat.

13. Verwendung eines Implantates gemäß einem der Ansprüche 1 bis 11 zur Verringerung der Dosis und/oder Dauer der Verabreichung einer systemischen Begleitmedikation mit ein oder mehreren blutgerinnungshemmenden Wirkstoffen vor, während und/oder nach Implantierung in einem menschlichen oder tierischen Organismus.

14. Verfahren zur Verringerung der Dosis und/oder Dauer der Verabreichung einer systemischen Begleitmedikation mit ein oder mehreren blutgerinnungshemmenden Wirkstoffen vor, während und/oder nach Implantierung eines Implantates in einen menschlichen oder tierischen Organismus, **dadurch gekennzeichnet, dass** ein Implantat gemäß einem der Ansprüche 1 bis 11 in den menschlichen oder tierischen Organismus implantiert wird.


Sequenzprotokoll:

<110> Biotronik VI Patent AG

<120> Implantat umfassend eine Oberfläche mit verringerten thrombogenen
Eigenschaften

<130> 07.171P-EP

<160> 4

<170> PatentIn version 3.4

<210> 1
<211> 9
<212> PRT
<213> unknown

<220>
<223> human

<400> 1

Cys Asp Pro Gly Tyr Ile Gly Ser Arg
1               5

<210> 2
<211> 19
<212> PRT
<213> unknown

<220>
<223> human

<400> 2

Cys Ser Arg Ala Arg Lys Gln Ala Ala Ser Ile Lys Val Ala Val Ser
1               5                   10                  15

Ala Asp Arg

<210> 3
<211> 4
<212> PRT
<213> unknown

<220>
<223> human

<220>
<221>  MISC_FEATURE
<222>  (1)..(4)
<223>  Pro in position 4 is an amide.

<400>  3

Gly Pro Arg Pro
1


<210>  4
<211>  4
<212>  PRT
<213>  unknown

<220>
<223>  human

<400>  4

Gly Pro Arg Pro
1

Sequenzprotokoll:

<110> Biotronik VI Patent AG

<120> Implantat umfassend eine Oberfläche mit verringerten thrombogenen
Eigenschaften

<130> 07.171P-EP

<160> 4

<170> PatentIn version 3.4

<210> 1
<211> 9
<212> PRT
<213> unknown

<220>
<223> human

<400> 1

Cys Asp Pro Gly Tyr Ile Gly Ser Arg
1               5

<210> 2
<211> 19
<212> PRT
<213> unknown

<220>
<223> human

<400> 2

Cys Ser Arg Ala Arg Lys Gln Ala Ala Ser Ile Lys Val Ala Val Ser
1               5                   10                  15

Ala Asp Arg

<210> 3
<211> 4
<212> PRT
<213> unknown

<220>
<223> human

<220>
<221>  MISC_FEATURE
<222>  (1)..(4)
<223>  Pro in position 4 is an amide.

<400>  3

Gly Pro Arg Pro
1

<210>  4
<211>  4
<212>  PRT
<213>  unknown

<220>
<223>  human

<400>  4

Gly Pro Arg Pro
1

Sequenzprotokoll:

<110> Biotronik VI Patent AG

<120> Implantat umfassend eine Oberfläche mit verringerten thrombogenen
Eigenschaften

<130> 07.171P-EP

<160> 4

<170> PatentIn version 3.4

<210> 1
<211> 9
<212> PRT
<213> homo sapiens

<400> 1

Cys Asp Pro Gly Tyr Ile Gly Ser Arg
1               5

<210> 2
<211> 19
<212> PRT
<213> Künstliche Sequenz

<220>
<223> note="Beschreibung der künstlichen Sequenz: Peptidsequenz basiert auf Peptid-
sequenz aus homo sapiens."

<400> 2

Cys Ser Arg Ala Arg Lys Gln Ala Ala Ser Ile Lys Val Ala Val Ser
1               5                   10                  15

Ala Asp Arg

<210>  3
<211>  4
<212>  PRT
<213>  homo sapiens

<220>
<221>  AMIDATION
<222>  (4)..(4)
<223>

<400>  3

Gly Pro Arg Pro
1


<210>  4
<211>  4
<212>  PRT
<213>  homo sapiens

<400>  4

Gly Pro Arg Pro
1